# EUROPEAN PATENT APPLICATION

(11) **EP 4 759 229 A1**
(43) Date of publication of application: **17.06.2026**
(21) Application number: 25198735.0
(22) Date of filing: 28.08.2025
(51) Int. Cl.: A61B 5/03, A61B 5/22, A61B 5/00

(54) **ORAL DETECTION DEVICE**

(30) Priority: 13.12.2024 TW 113148618
(71) Applicant: Yuh & Jih Industrial Co., Ltd., 407019 Taichung City (TW)
(72) Inventor: TU, Guo-Xian, 407019 Taichung City (TW); TU, I-Hsuan, 407019 Taichung City (TW)
(74) Representative: Mewburn Ellis LLP

(57) **Abstract**

A oral detection device includes a case unit (1), a detection unit (2), an inflator unit (3), a connector (4), a pressure sensor (5), and a control unit (7). The detection unit (2) includes a gas bag (21) adapted to be put into an oral cavity of a subject and defining a gas chamber (211). The inflator unit (3) includes a main body (31) and an inflator tube (32) having a connection member (323). The pressure sensor (5) is to detect pressure in the gas chamber (211) so as to generate a pressure detection value. The control unit (7) is for controlling the inflator unit (3) to fill the gas chamber (211) with gas, and for comparing the pressure detection value received from the pressure sensor (5) with a pressure standard value to generate a detection result regarding an oral function of the subject.

## Description

The disclosure relates to a detection device, and more particularly to an oral detection device.

Functions of human organs may decrease with age, such as difficulty in swallowing due to loss of muscle strength. The above symptom is not easily found and usually requires diagnosis by medical personnel with specific instruments in clinics or hospitals. It is inconvenient for elderly patients to frequently go to medical institutions, which not only wastes time and effort but also increases the risk of contracting infectious diseases; thus improvements are needed.

Therefore, an object of the disclosure is to provide an oral detection device that can alleviate at least one of the drawbacks of the prior art.

According to an aspect of the disclosure, there is provided an oral detection device according to claim 1.

Other features and advantages of the disclosure will become apparent in the following detailed description of the embodiment(s) with reference to the accompanying drawings. It is noted that various features may not be drawn to scale.
Figure 1 is a fragmentary partly-exploded perspective view of an embodiment of an oral detection device according to the present disclosure.
Figure 2 is a fragmentary perspective view of the embodiment.
Figure 3 is an exploded perspective view illustrating a gas bag and a bite tube of a detection unit of the embodiment.
Figure 4 is an exploded sectional view of Figure 3.
Figure 5 is a sectional view of the gas bag and the bite tube.
Figure 6 is an exploded sectional view illustrating a connector of the embodiment.
Figure 7 is a sectional view of the connector.
Figure 8 is a fragmentary exploded sectional view, illustrating the connector and an inflator unit of the embodiment.
Figure 9 is a top view of the connector.
Figure 10 is a sectional view taken along line X-X in Figure 8.
Figure 11 is a fragmentary schematic sectional view, illustrating the connector being connected to the inflator unit.
Figure 12 is a view similar to Figure 11.
Figure 13 is a view similar to Figure 8.
Figure 14 is a fragmentary schematic view illustrating the connector being operated to be separated from the inflator unit.
Figure 15 is a block diagram of illustrating the inflator unit, a control unit, a pressure sensor and a display unit of the embodiment.

Before the disclosure is described in greater detail, it should be noted that where considered appropriate, reference numerals or terminal portions of reference numerals have been repeated among the figures to indicate corresponding or analogous elements, which may optionally have similar characteristics.

It should be noted herein that for clarity of description, spatially relative terms such as "top," "bottom," "upper," "lower," "on," "above," "over," "downwardly," "upwardly" and the like may be used throughout the disclosure while making reference to the features as illustrated in the drawings. The features may be oriented differently (e.g., rotated 90 degrees or at other orientations) and the spatially relative terms used herein may be interpreted accordingly.

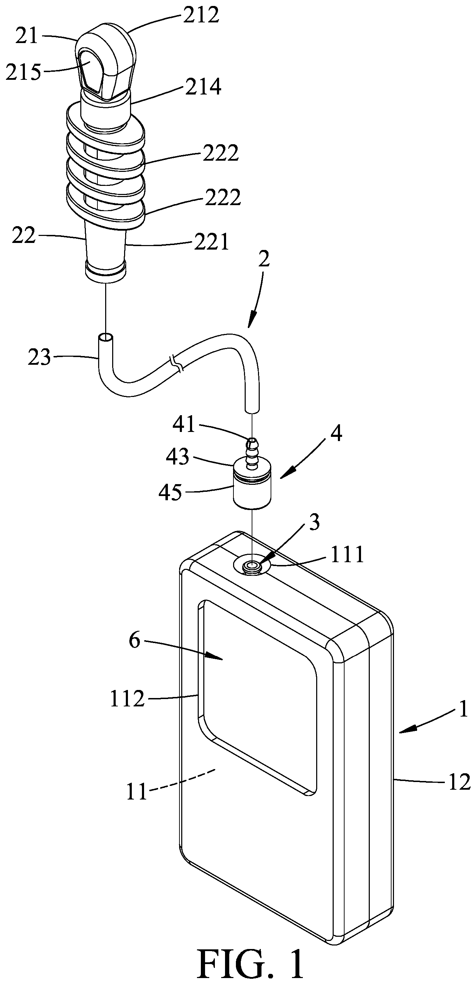

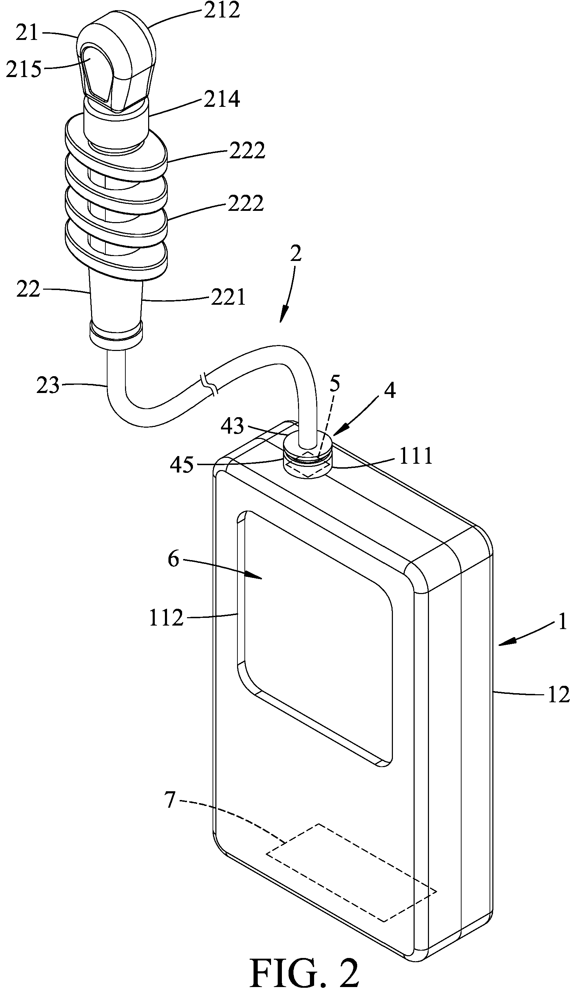

Referring to Figures 1 and 2, an embodiment of an oral detection device according to the disclosure is used for detecting functions of oral cavity muscles. The oral detection device includes a case unit 1, a detection unit 2, an inflator unit 3, a connector 4, a pressure sensor 5, a display unit 6, and a control unit 7.

The case unit 1 includes a case body 12 that defines an accommodation space 11. The case unit 1 further has an installation hole 111 that is formed in a top side of the case body 12 and that is in spatial communication with the accommodation space 11 and an external environment, and a front groove 112 that is formed in a front side of the case body 12.

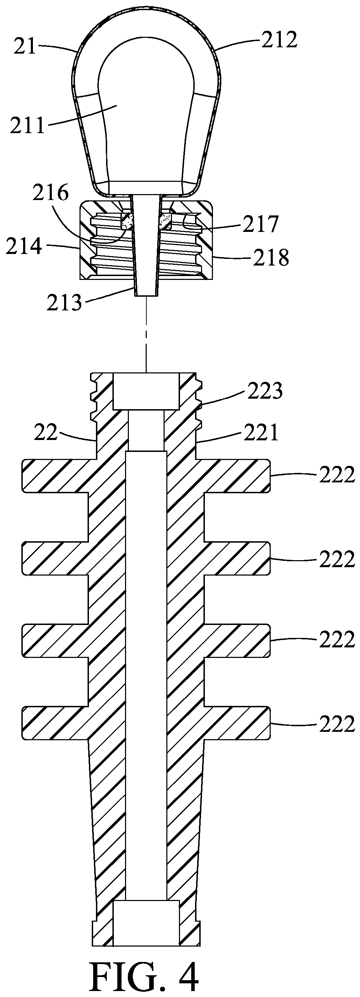

The detection unit 2 is disposed on the case unit 1, and includes a gas bag 21 that is adapted to be put into an oral cavity of a subject (e.g., a patient) (not shown) and that defines a gas chamber 211 (see Figure 4), a connection tube 23 that is in gas communication with the gas bag 21, and a bite tube 22 that is connected between the gas bag 21 and the connection tube 23, that is adapted for teeth of the subject to bite, and that is made of a resilient material.

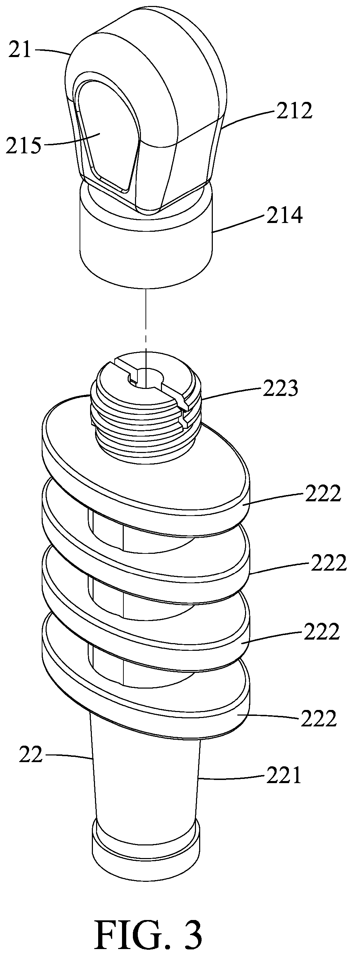

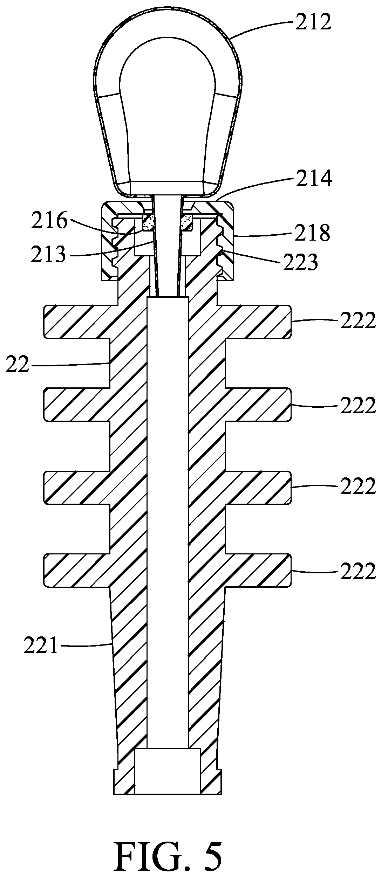

Referring to Figures 3 to 5, the gas bag 21 has a bag body 212 that defines the gas chamber 211, an extension tube 213 that extends from the bag body 212 into the bite tube 22, and a screw connection member 214 that surrounds the extension tube 213. The bag body 212 of the gas bag 21 has a recess groove 215 that is recessed inward from an outer surface of the gas bag 21. In practice, when the gas bag 21 is put into the oral cavity of the patient, the patient has to abut their tongue upward against the gas bag 21 and the tongue can be received in the recess groove 215, which provides better comfort for the patient. The extension tube 213 has a flange portion 216. The screw connection member 214 has a limit wall 217 that is located between the flange portion 216 and the bag body 212 and through which the extension tube 213 extends, and a screw connection ring 218 that extends downward from a periphery of the limit wall 217.

The bite tube 22 has a tube body 221 that extends outwards from the gas bag 21, and a plurality of separation rings 222 that are spaced apart from each other and that are formed on the tube body 221. The tube body 221 has a screw connection segment 223 that is screwed to the screw connection member 214. In this embodiment, the bite tube 22 is made of, for example, but not limited to, silicone, which increases comfort. In practice, by virtue of containing one or more separation rings 222 in the oral cavity (i.e., biting a space between any adjacent two of the separation rings 222), the gas bag 21 is difficult to drop out of the oral cavity, which increases stability of use. The screw connection ring 218 is screwed to the screw connection segment 223 of the bite tube 22.

In this embodiment, the gas bag 21 is separably connected to the tube body 221 of the bite tube 22, which is convenient for cleaning and replacement of the bag body 212 and the bite tube 22 and for bag bodies 212 and bite tubes 22 having different sizes; but in other embodiments, the gas bag 21 and the bite tube 22 may be formed in one-piece.

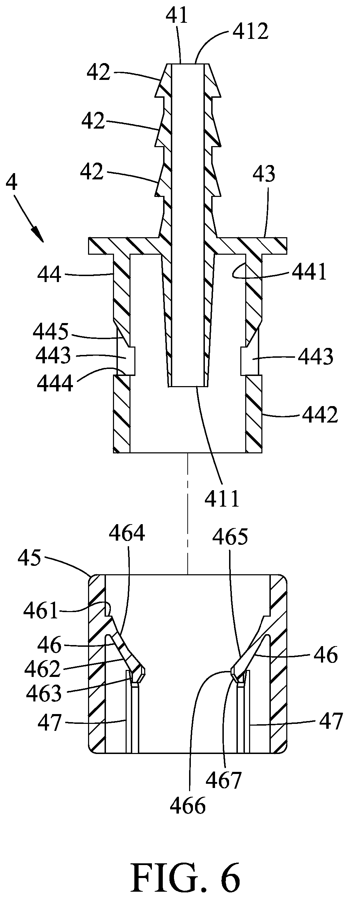

Referring to Figures 6 to 8, the inflator unit 3 is disposed in the case unit 1 (specifically, in the accommodation space 11), and includes a main body 31, and an inflator tube 32 that is connected to the main body 31, and that extends outward from the main body 31 to be secured in the installation hole 111. The main body 31 may be embodied as an electric pump, but is not limited thereto. The inflator tube 32 has an inner end 321 that is connected to the main body 31, an outer end 322 that is opposite to the inner end 321, and a connection member 323 that extends radially outwardly from the outer end 322. The outer end 322 of the inflator tube 32 has therein a plurality of diaphragms 324 that are made of a resilient material.

In this embodiment, the connection member 323 is a protrusion, and has an inner end edge 325 that is rounded, an outer end edge 326 that is rounded and that is opposite to the inner end edge 325, and a side surrounding surface 327 that is connected between the inner end edge 325 and the outer end edge 326. In some examples, the connection member 323 may be configured as an annular protrusion.

The connector 4 is separably connected between the inflator tube 32 of the inflator unit 3 and the connection tube 23 of the detection unit 2. The inflator unit 3 is operable for filling the gas chamber 211 of the gas bag 21 with gas through the connector 4. By virtue of the connector 4 being able to be quickly separated from or connected to the inflator unit 3 and the detection unit 2 (details will be described in the following), storage, cleaning and carrying of the oral detection device are convenient; when the detection unit 2 is damaged or aged, replacement thereof is easy. In this embodiment, the connector 4 includes a communication tube 41, a plurality of conical rings 42, a stop wall 43, an inner ring 44, an outer ring 45, at least one abutment member 46, and a plurality of limitation ribs 47. The at least one abutment member 46 includes a plurality of abutment members 46 (in this embodiment, includes two abutment members 46). The outer ring 45 is formed with the abutment members 46. In this embodiment, each of the abutment members 46 is a limit arm, and is made of a resilient material.

The communication tube 41 has an inflation end 411 that is connected to the inflator tube 32 of the inflator unit 3, and a communication end 412 that is connected to the connection tube 23 of the detection unit 2.

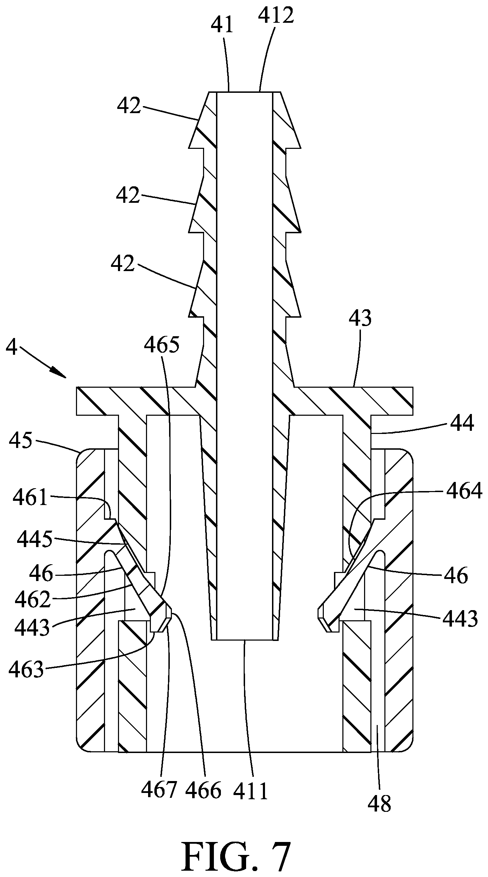

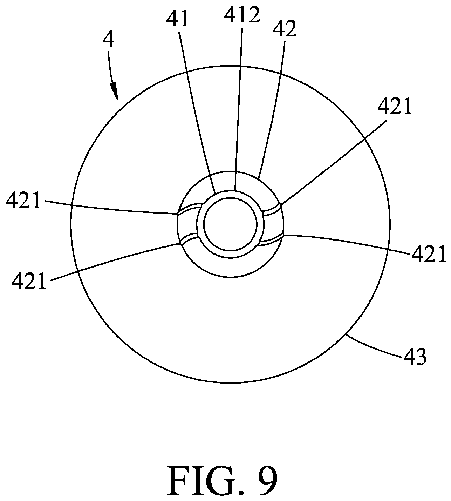

Referring to Figures 7 to 9, the conical rings 42 are spaced apart from each other and are sleeved on the communication end 412 of the communication tube 41, and one of the conical rings 42 that is furthest from the inflation end 411 (i.e., that is adjacent to the communication end 412 the most) has a plurality of curved grooves 421, which helps the communication end 412 be rotated and thus be inserted into the connection tube 23 easily. The stop wall 43 is located between the inflation end 411 and the communication end 412, and is for abutment against the connection tube 23.

Referring to Figures 6 to 8, the inner ring 44 is connected to the stop wall 43 and surrounds the inflation end 411. The inner ring 44 has an inner surrounding surface 441, an outer surrounding surface 442, and a plurality of extension grooves 443 that extend through the inner surrounding surface 441 and the outer surrounding surface 442. In this embodiment, there are two extension grooves 443. Each of the extension grooves 443 is defined by a groove surrounding surface 444, and each of the groove surrounding surfaces 444 has an oblique portion 445 that extends obliquely relative to the inner surrounding surface 441.

The inner ring 44 is located between the outer ring 45 and the inflation end 411 of the communication tube 41; specifically, the inner ring 44 is surrounded by the outer ring 45 and surrounds the inflation end 411, and the outer ring 45 is movable relative to the inner ring 44.

The abutment members 46 are spaced apart from the inflation end 411, extend obliquely from an inner side of the outer ring 45 towards the main body 31, and engage the connection member 323 of the inflator tube 32. The abutment members 46 and the outer ring 45 are made of a resilient material and are formed in one-piece. The abutment members 46 respectively extend through and are movable in the extension grooves 443, and are resiliently pushable by the connection member 323 of the inflator tube 32. Each of the abutment members 46 has a first end 461 that is connected to the inner side of the outer ring 45, a swing arm portion 462 that obliquely extends from the first end 461 towards the main body 31, and a second end 463 that extends from the swing arm portion 462 towards the main body 31.

Each of the abutment members 46 further has a contact surface 464 that includes a first oblique surface 465 extending obliquely towards the main body 31 and facing a respective one of the oblique portions 445, a connection surface 466 extending from the first oblique surface 465 towards the main body 31 and operable for resiliently abutting against the connection member 323, and a second oblique surface 467 extending obliquely from the connection surface 466 towards the outer ring 45.

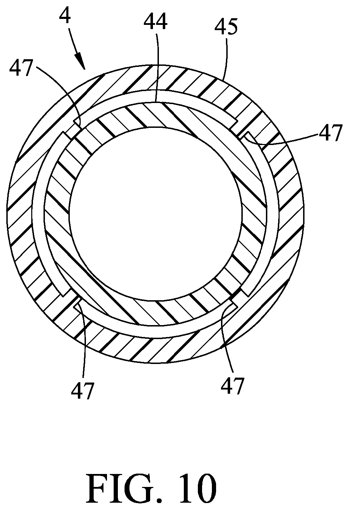

Referring to Figures 7, 8 and 10, the inner ring 44 and the outer ring 45 cooperatively define a gap 48 therebetween. The limitation ribs 47 are located in the gap 48, and are formed on the inner side of the outer ring 45 for abutment against the inner ring 44.

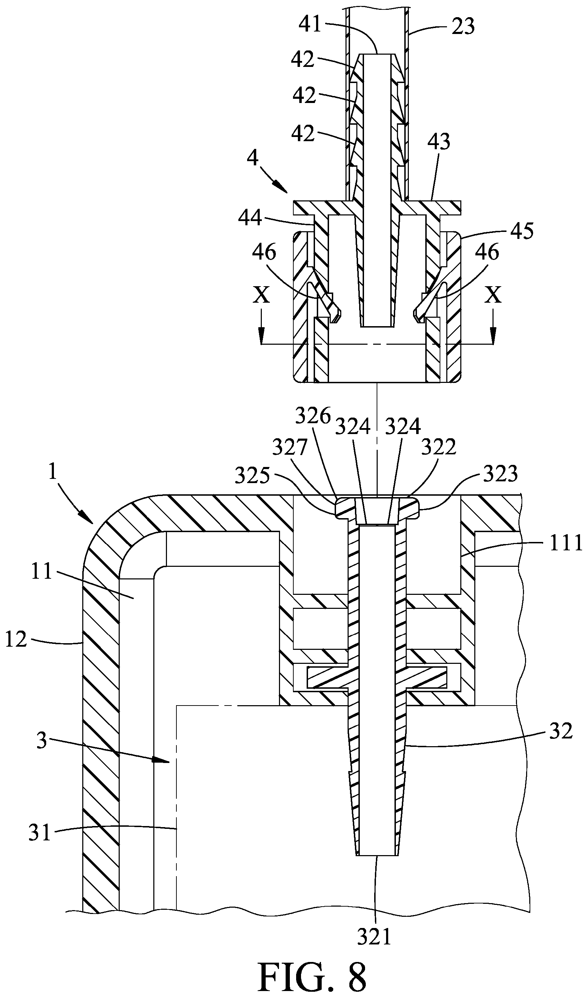

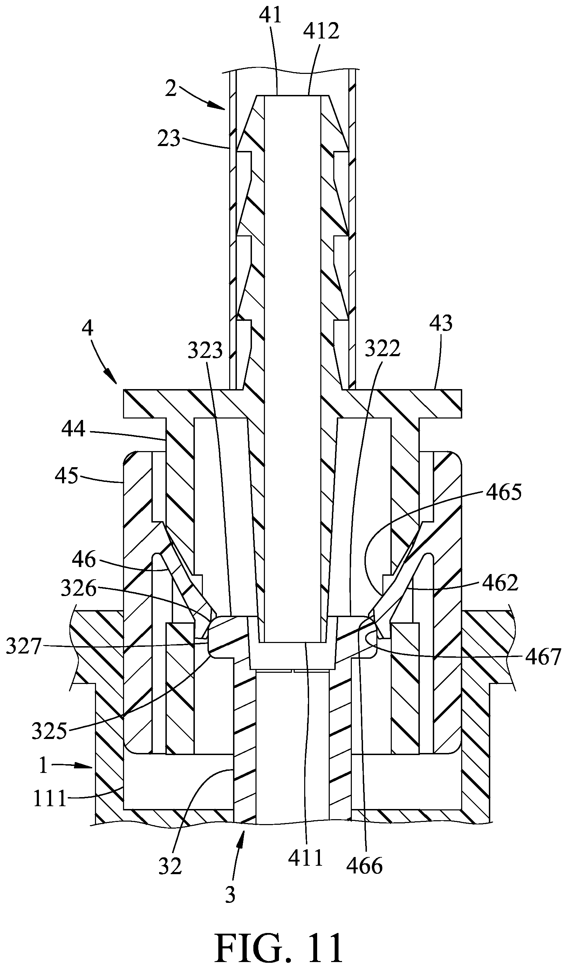

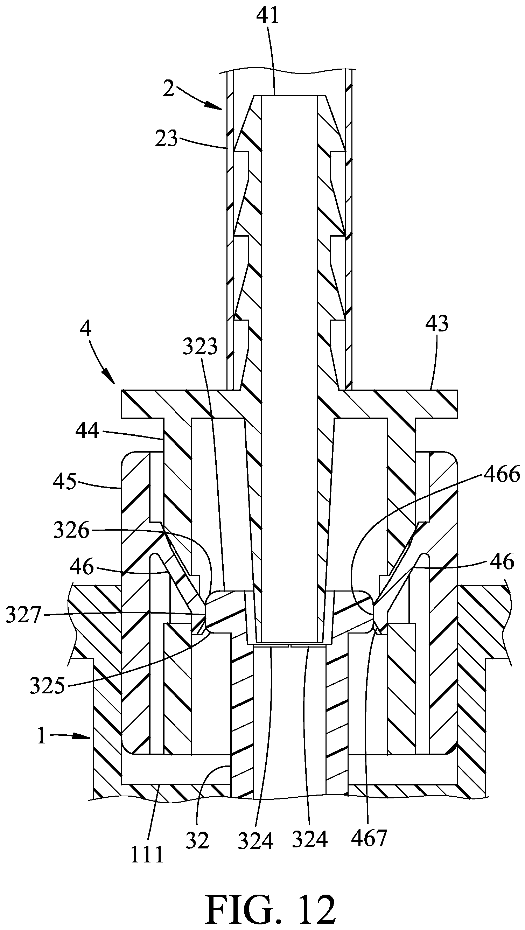

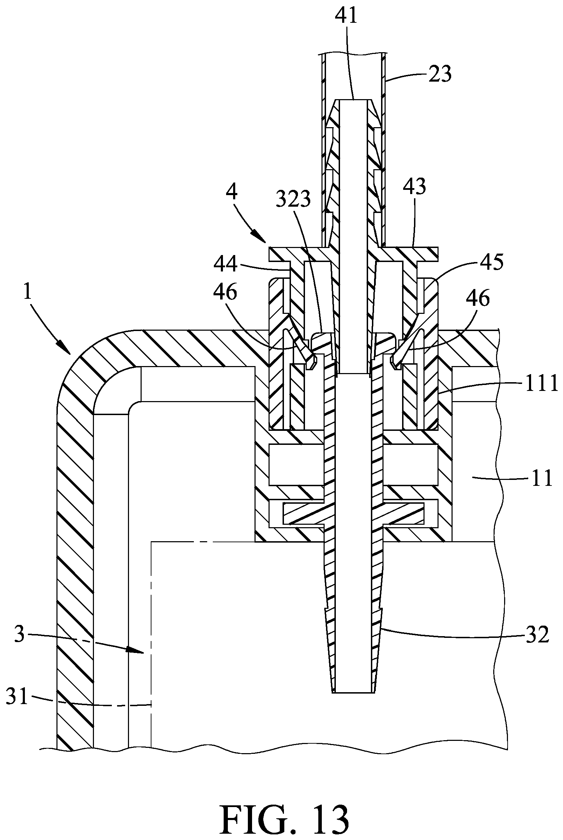

Referring to Figures 8, and 11 to 13, when the detection unit 2 is being connected to the inflator unit 3, the connector 4, along with the connection tube 23 of the detection unit 2, is put into the installation hole 111, and the second oblique surface 467 of each of the abutment members 46 first contacts the outer end edge 326 of the connection member 323 (see Figure 11). Then, as shown in Figure 12, the connection surface 466 of each of the abutment members 46 contacts the side surrounding surface 327 and is resiliently pushed by the connection member 323, so that the abutment members 46 swing outward, and at the same time, the inflation end 411 of the communication tube 41 pushes the diaphragms 324 away to extend into and be connected to the inflator tube 32. As shown in Figure 13, the first oblique surface 465 of each of the abutment members 46 engages the connection member 323 (specifically, engages the inner end edge 325), so that the communication tube 41 of the connector 4 and the inflator tube 32 are airtightly connected to each other.

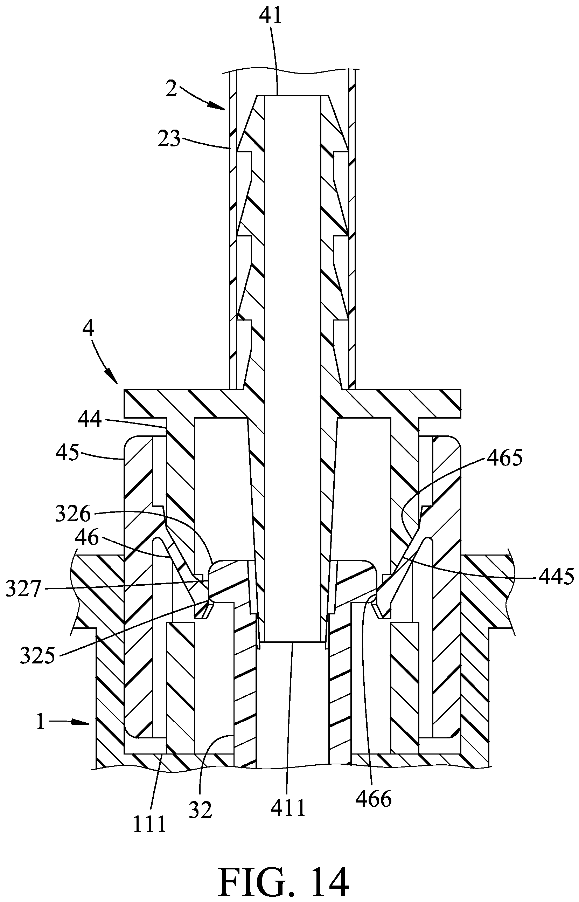

Referring to Figures 13 and 14, when the detection unit 2, along with the connector 4, is being separated from the inflator unit 3, the outer ring 45 is pulled upward (see Figure 14), so that the first oblique surface 465 of each of the abutment members 46 contacts and slides on the respective oblique portion 445, and the connector 4 is able to be separated from the inflator unit 3 when the first oblique surface 465 of each of the abutment members 46 is disengaged from the inner end edge 325. This embodiment has advantages of small detachment force and labor-saving. In other embodiments, the inner ring 44 may be omitted; for example, when the connector 4 is embodied as an one-piece inflator head, the abutment members 46 are configured as internal threads and the connection member 323 of the inflator tube 32 is configured as external threads, which also achieves the function of mounting/separating the connector 4 to/from the inflator tube 32.

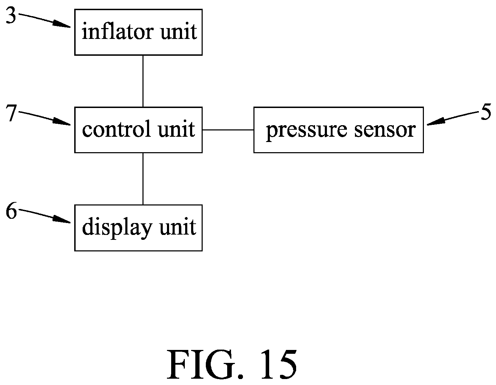

Referring to Figures 2 and 15, the pressure sensor 5 is disposed on the case unit 1 or the connector 4 and is configured to detect pressure in the gas chamber 211 so as to generate a pressure detection value. In this embodiment, the pressure sensor 5 is disposed on the case unit 1. The pressure sensor 5 may be a gas pressure pressure sensor, but is not limited to this aspect. The display unit 6 is disposed in the front groove 112 of the case unit 1, and may be a touch screen, but is not limited thereto.

The control unit 7 is disposed in the case unit 1 and is signally connected to the main body 31 of the inflator unit 3, the pressure sensor 5 and the display unit 6. The control unit 7 is operable for controlling the inflator unit 3 to fill the gas chamber 211 with the gas, and for comparing the pressure detection value received from the pressure sensor 5 with a pressure standard value to thereby generate a detection result regarding an oral function of the subject (e.g., related to the functions of oral cavity muscles). The display unit 6 is adapted to display the detection result. Through the detection result, the functions of oral cavity muscles may be determined; for example, if the pressure detection value (e.g., a value of tongue pressure) is found to be low, the patient may have difficulty in swallowing or snoring. The control unit 7 may be a printed circuit board module having a microprocessor, but is not limited thereto. In practice, the oral detection device may be powered by a rechargeable battery, so the oral detection device is convenient to carry and not limited to user sites. In addition, in some embodiments, the control unit 7 may be equipped with a built-in wireless transmission module, so the detection may be activated by a mobile device, and the detection result may be displayed on the mobile device (in this example, the display unit 6 may be omitted). Since the rechargeable battery and the connection of the mobile device are existing techniques, details thereof will be omitted herein for the sake of brevity.

By virtue of the abutment members 46 of the connector 4 engaging the connection member 323 of the inflator tube 32, the detection unit 2 is able to be quickly connected to the inflator unit 3 before the inflator unit 3 fills the gas bag 21 with gas. During the detection, the patient puts the gas bag 21 into their oral cavity, their tongue abuts against and presses the gas bag 21 upward to squeeze the gas therein, then through the pressure sensor 5, the pressure in the gas chamber 211 is sensed, and finally the detection result is acquired through the control unit 7 after comparison. The detection result may include, but not limited to, the pressure detection value, and indication of "being lower than the pressure standard value," "suggesting self-training," or "suggesting hospital treatment" to show whether the pressure detection value is greater than the the pressure standard value and to thereby acknowledge the functions of oral cavity muscles. The disclosure is easy to operate and facilitates self-detection for the general public, which is convenient and may be used often. In addition, the separable design of the detection unit 2 and the inflator unit 3 can reduce storage space, and thus the oral detection device is easy to carry; therefore, the object of the disclosure can indeed be achieved.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. An oral detection device, **characterized by**:
a case unit (1);
a detection unit (2) disposed on said case unit (1) and including a gas bag (21) that is adapted to be put into an oral cavity of a subject and that defines a gas chamber (211), and a connection tube (23) that is in gas communication with said gas bag (21);
an inflator unit (3) disposed in said case unit (1) and including a main body (31) and an inflator tube (32) that is connected to said main body (31), and that has a connection member (323);
a connector (4) separably connected between said inflator unit (3) and said connection tube (23) of said detection unit (2), said inflator unit (3) being operable for filling said gas chamber (211) of said gas bag (21) with gas through said connector (4), said connector (4) including a communication tube (41) and at least one abutment member (46), said communication tube (41) having an inflation end (411) that is connected to said inflator tube (32) of said inflator unit (3), and a communication end (412) that is connected to said connection tube (23) of said detection unit (2), said at least one abutment member (46) being spaced apart from said inflation end (411) and engaging said connection member (323) of said inflator tube (32);
a pressure sensor (5) configured to detect pressure in said gas chamber (211) so as to generate a pressure detection value; and
a control unit (7) disposed on said case unit (1) and signally connected to said inflator unit (3) and said pressure sensor (5), said control unit (7) being operable for controlling said inflator unit (3) to fill said gas chamber (211) with the gas, and for comparing the pressure detection value received from said pressure sensor (5) with a pressure standard value to thereby generate a detection result regarding an oral function of the subject.

2. The oral detection device as claimed in claim 1, wherein:
said connection member (323) of said inflator tube (32) is a protrusion, and said at least one abutment member (46) includes a plurality of abutment members (46);
each of said plurality of abutment members (46) is a limit arm, is made of a resilient material, and has a first oblique surface (465) that extends obliquely towards said main body (31), and a connection surface (466) that extends from said first oblique surface (465) towards said main body (31) and that is operable for resiliently abutting against said connection member (323); and
said first oblique surface (465) of each of said plurality of abutment members (46) engages said connection member (323) so that said connector (4) is connected to said inflator tube (32) of said inflator unit (3).

3. The oral detection device as claimed in claim 2, wherein said connector (4) further includes an outer ring (45) and an inner ring (44), said outer ring (45) being connected to said plurality of abutment members (46), said inner ring (44) being located between said outer ring (45) and said communication tube (41), said inner ring (44) having a plurality of extension grooves (443), said plurality of abutment members (46) respectively extending through and being movable in said plurality of extension grooves (443).

4. The oral detection device as claimed in any one of claims 1 to 3, wherein said inflator tube (32) of said inflator unit (3) further has a plurality of diaphragms (324) that are made of a resilient material, said inflation end (411) of said connector (4) pushing said plurality of diaphragms (324) away to extend into and be connected to said inflator tube (32) so that said communication tube (41) and said inflator tube (32) are airtightly connected to each other.

5. The oral detection device as claimed in any one of claims 1 to 4, wherein said connector (4) further includes a plurality of conical rings (42) that are spaced apart from each other and that are sleeved on said communication end (412) of said communication tube (41), and one of said plurality of conical rings (42) that is furthest from said inflation end (411) has a plurality of curved grooves (421).

6. The oral detection device as claimed in any one of claims 1 to 5, further comprising a display unit (6) that is disposed on said case unit (1) and that is adapted to display the detection result, wherein:
said detection unit (2) further includes a bite tube (22) that is connected between said gas bag (21) and said connection tube (23), that is adapted for teeth of the subject to bite, and that is made of a resilient material.

7. The oral detection device as claimed in claim 6, wherein said bite tube (22) has a tube body (221) that extends outwards from said gas bag (21), and a plurality of separation rings (222) that are spaced apart from each other and that are formed on said tube body (221).

8. The oral detection device as claimed in claim 7, wherein said gas bag (21) is separably connected to said bite tube (22) and has a screw connection member (214), and said tube body (221) has a screw connection segment (223) that is screwed to said screw connection member (214).

9. The oral detection device as claimed in claim 8, wherein said gas bag (21) has a bag body (212) that defines said gas chamber (211), and an extension tube (213) that extends from said bag body (212) into said bite tube (22), said screw connection member (214) being connected to said extension tube (213), and having a limit wall (217) through which said extension tube (213) extends, and a screw connection ring (218) that extends from a periphery of said limit wall (217) and that is screwed to said screw connection segment (223) of said bite tube (22).

10. The oral detection device as claimed in any one of claims 1 to 9, wherein said gas bag (21) has a recess groove (215) that is recessed inward from an outer surface of said gas bag (21).
